Europäisches Patentamt

(19) European Patent Office

Office européen des brevets

(11) Numéro de publication: **0 024 256**
**A2**

(12) **DEMANDE DE BREVET EUROPEEN**

(21) Numéro de dépôt: **80810243.8**

(22) Date de dépôt: **01.08.80**

(51) Int. Cl.³: **A 61 B 17/18**

---

(30) Priorité: **08.08.79 CH 7268/79**

(43) Date de publication de la demande:
**25.02.81 Bulletin 81/8**

(84) Etats Contractants Désignés:
**AT BE DE FR GB IT NL SE**

(71) Demandeur: **JF ORTHOPEDIE S.A.**
**Schlossbergstrasse 22**
**CH-8702 Zollikon(CH)**

(72) Inventeur: **Jaquet, Henri**
**Chemin de Tavernay 8**
**CH-1218 Le Grand-Saconnex(CH)**

(74) Mandataire: **Dietlin, Henri et al,**
**DIETLIN, MOHNHAUPT & Cie 3, quai du Mont-Blanc**
**CH-1201 Genève(CH)**

(54) Appareil d'allongement et de compression pour fixation squelettique externe.

㊼ L'appareil d'allongement et de compression pour fixation squelettique externe comprend une partie centrale (1) d'allongement et de compression et deux parties d'extrémité (2,3) agencées pour retenir des tiges 5 supportant des étaux 6 de fixation pour des fiches transcutanées.

La partie centrale (1) comprend une pièce d'avance (27) se présentant sous forme d'un demi-cylindre coulissant contre un deuxième demi-cylindre, le tout étant placé à l'intérieur d'un cylindre extérieur (20).

EP 0 024 256 A2

./...

Fig. 1.

- 1 -

## Appareil d'allongement et de compression
## pour fixation squelettique externe

L'invention a pour objet un appareil d'allongement pour fixation squelettique externe, notamment pour fixation par fiches transcutanées.

Il existe déjà sur le marché des appareils d'allongement pour fixation squelettique externe. Ces appareils sont en général constitués de tiges habituellement filetées sur lesquelles viennent se visser les éléments de maintien des fiches transcutanées. Ces types d'appareils offrent de nombreux désavantages. Le principal désavantage consiste dans le fait que les moyens de commande de l'allongement ou de la compression ne sont pas indépendant des moyens d'assemblage de l'ensemble. En d'autres mots, lorsque l'on veut faire varier l'allongement ou la compression, on risque de dérégler complètement la mise en place des éléments de l'appareil. Comme ces appareils sont destinés à maintenir des fragments d'os cassés sur un patient, un tel risque n'est pas acceptable. Ces appareils n'ont d'autre part pas de possibilité de blocage sûre, de sorte qu'il y a risque qu'une bague d'avance se déserre ou tourne, étant donné que ces bagues sont en général apparentes sur la tige filetée.

Il a également été proposé des appareils constitués de tubes coulissants de section carrée ou cylindrique. Ces types d'appreils présentent les mêmes désavantages que les appareils à tiges filetées. D'autre part, ces appareils ne forment pas une structure rigide et ils présentent souvent du jeu, de sorte que les allongements ou les compression ne peuvent être exécutés avec précision. Les appareils à tube, comme les appareils à tige filetée présentent encore des moyens de fixation des fiches à possibilités limitées, c'est-à-dire qui ne permettent pas toujours de positionner les fiches comme l'utilisateur le désirerait. Souvent, les moyens de fixation des fiches ne permettent qu'un déplacement selon une direction ou selon une place.

Le but de l'invention est de remédier aux inconvénients mentionnés ci-dessus et de proposer un appareil d'allongement ou de compression pour fixation squelettique parfaitement rigide et permettant d'effectuer un allongement ou une compression avec précision sans dérégler le positionnement des pièces d'assemblage de l'appareil. L'appareil présentera encore l'avantage de pouvoir venir se fixer sur des fiches ou des éléments placés dans n'importe quelle position.

L'appareil selon l'invention présente des moyens d'assemblage de fiches transcutanées ou d'éléments reliés à ces fiches et des moyens d'allongement et de compression placés entre les moyens d'assemblage et est caractérisé en ce que les moyens d'allongement et de compression comprennent au moins deux pièces coulissantes l'une par rapport à l'autre selon un axe commun, l'une des pièces présentant un filetage attaqué par un filetage correspondant d'un anneau d'avance maintenu dans le sens de l'axe d'avance sur les moyens d'allongement et de compression, des moyens de rattrapage de jeu étant prévus entre les deux pièces.

Selon un mode d'exécution préféré, les deux pièces coulissantes comprennent un demi-cylindre d'avance fileté et un demi-cylindre d'appui placés face à face, le tout étant maintenu à l'intérieur d'un cylindre externe, et le demi-cylindre d'appui comprend une fente s'étendant sur une partie de sa longueur et séparant le demi-cylindre en deux parties, au moins une vis de rattrapage de jeu et une vis de blocage étant introduites dans des alésages filetés correspondants prévus dans une des parties du demi-cylindre et débouchant sur l'autre partie.

Les deux demi-cylindres d'avance et d'appui présentent respectivement une rainure de section rectangulaire et une gorge de section circulaire placées adjacentes, de manière à laisser un vide sur l'axe commun des deux demi-cylindres, une règle graduée étant placée au fond de la rainure du cylindre d'avance.

Les moyens d'assemblage comprennent deux éléments de blocage agencés pour bloquer une tige dans n'importe quelle direction, les tiges bloquées sur les éléments de blocage étant montées sur une des mâchoires d'un étau de fixation des fiches transcutanées maintenant les fragments d'os.

Les éléments de blocage orientables peuvent présenter deux pièces latérales serrant entre elles deux plaquettes circulaires tournant entre les pièces latérales et dont les surfaces adjacentes présentent chacune une rainure diamétrale apte à serrer une tige, les pièces latérales étant retenues à une de leurs extrémités dans une gorge d'une cuvette reliée à l'extrémité de la pièce coulissante, respectivement du cylindre externe, les deux autres extrémités des pièces latérales étant traversées par une vis de serrage.

L'appareil est réalisé en un alliage de titane ou d'aluminium ou en acier inox.

Le dessin représente, à titre d'exemple, un mode de réalisation d'un appareil d'allongement et de compression pour fixation squelettique externe.

Dans le dessin:

La fig. 1 représente une vue en perspective du mode de réalisation de l'appareil,

la fig. 2 représente une coupe longitudinale de la partie centrale de l'appareil,

la fig. 3 est une coupe transversale selon la ligne III-III de la fig. 2,

la fig. 4 est une coupe transversale selon la ligne IV-IV de la fig. 2,

la fig. 5 est une coupe transversale selon la ligne V-V de la fig. 2,

la fig. 6 est une variante d'une partie de l'appareil des fig. 1 à 5 permettant d'intercaler entre les moyens d'assemblage des fiches transcutanées un dynamomètre de mesure des forces d'allongement ou de compression appliquées sur les parties squelettiques maintenues par les fiches.

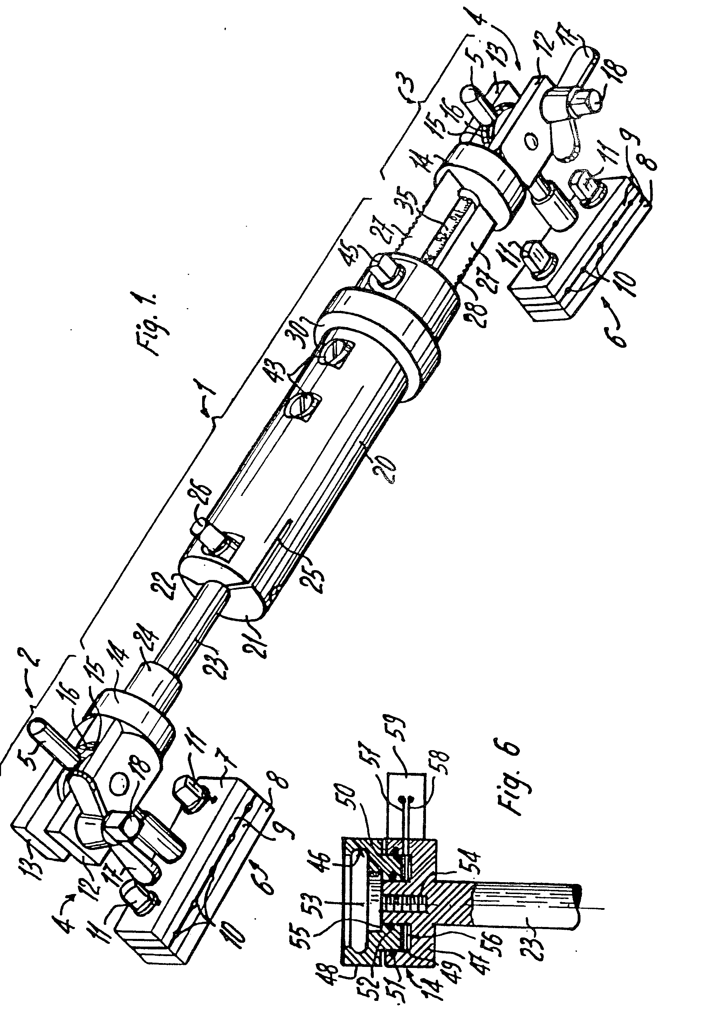

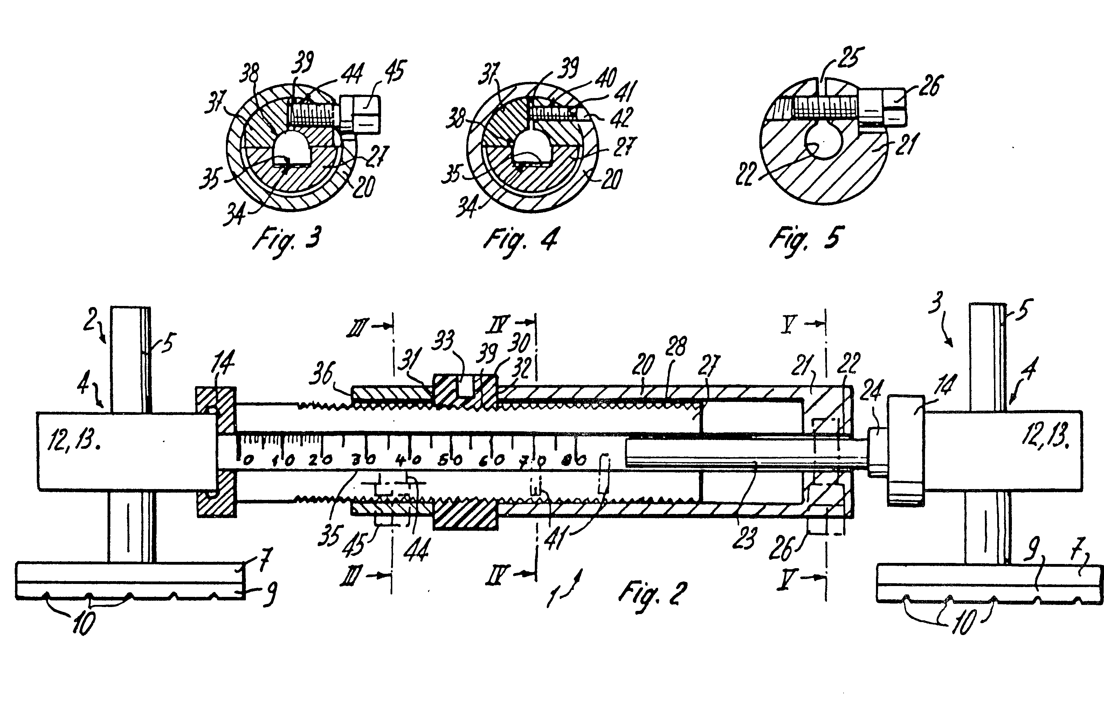

L'appareil d'allongement et de compression représenté dans les fig. 1 à 5 du dessin présente une partie centrale 1 et deux parties d'extrémité 2 et 3, chacune des parties 2 et 3 comprenant un élément de blocage orientable 4 destiné à recevoir une tige 5 et portant à une de ses extrémités un étau 6 formé de deux mâchoires 7 et 8. Les mâchoires 7 et 8 présentent des garnitures intérieures 9, formées de couches de tissu stratifié englobées dans une résine synthétique, dont les surfaces adjacentes en contact sont munies de rainures correspondantes 10 aptes à retenir entre chacun des étaux 6 de 1 à 5 fiches de fixation transcutanées non représentées. Les deux mâchoires 7 et 8 sont serrées à l'aide de vis à tête carrée 11 (voir en particulier fig. 1). Dans la fig. 2, les éléments de blocage orientables, appelés ci-après rotules de fixation, enserrant la tige 5 solidaire de la mâchoire supérieure 7 des étaux 6 (voir en particulier fig. 1) comprennent deux pièces latérales 12 et 13 retenues à une de leurs extrémités dans une gorge circulaire non visible sur le dessin placée à l'intérieur d'une cuvette 14 figurant les extrémités de la partie centrale 1 de l'appareil. Les pièces latérales 12 et 13 des rotules de fixation 4 serrent deux plaquettes circulaires 15, 16 (fig. 1) dont les surfaces intérieures adjacentes présentent une rainure s'étendant selon un diamètre de chacune des plaquettes, les tiges 5 supportant les mâchoires 7 étant introduites dans les rainures opposées des plaquettes 15, 16 et serrées par l'ensemble formé des pièces 12, 13, des plaquettes 15, 16 et de la rainure de la cuvette 14 au moyen de vis à ailettes 17 traversant l'extrémité libre de la pièce 12 pour venir se visser dans la pièce 13. Le serrage effectué par les vis à ailettes 17 permet de positionner et bloquer fermement les mâchoires 7 des étaux 6 dans la position adéquate désirée par l'utilisateur. En effet, les pièces latérales 12, 13 peuvent tourner d'un angle de 360° à l'intérieur de la gorge non représentée de la cuvette 14 et des plaquettes 15, 16 peuvent tourner également de 360° entre les pièces latérales 12 et 13. Finalement, la tige 5 peut tourner de 360° sur son axe dans les rainures adjacentes non représentées des plaquettes 15, 16. Il est ainsi clairement compréhensible pour l'homme du métier que l'étau 6 peut être placé dans n'importe quelle position. La vis à ailette 17 présente à sa partie supérieure une tête carrée 18 de même dimension que les têtes des vis de serrage 11 des mâchoires 7 et 8. Les rotules 4, de même que les mâchoires 7 et 8 peuvent ainsi être bloquées fermement à l'aide d'une clé non représentée agencée pour venir épouser les têtes des vis 11 ou des vis à ailettes 17. Les mâchoires 7, 8 des étaux 6, la tige 5 ainsi que tous les éléments constituants la rotule 4 seront de préférence réalisés en métal, par exemple en acier inox ou en un alliage léger, tel qu'un alliage d'aluminium. Les garnitures 9 des mâchoires 7 et 8 seront de préférence réalisées en matière plastique.

Comme mentionné plus haut, les rainures 10 prévues sur les surfaces intérieures adjacentes des garnitures sont destinées à recevoir des fiches transfixiantes non représentées. Ces fiches sont parfaitement connues de l'homme du métier et ne seront par conséquent pas décrites en détail. Il sera simplement mentionné que ce sont de longues tiges en acier inox de section cylindrique dont une extrémité se présente sous forme d'une pointe apte à creuser les tissus où elle doit s'enfoncer, l'autre extrémité présentant une tête susceptible de recevoir une clé ou un mandrin d'une perceuse ou d'un vilebrequin. Cette fiche ou tige peut présenter une partie centrale filetée. Elle est destinée à venir se fixer sur un os après avoir traversé la chair. Elle peut également traverser complètement un os et la chair qui le recouvre, de manière à présenter deux extrémités libres susceptibles d'être serrées par les rainures 10 des mâchoires 7 et 8 des étaux 6.

Comme mentionné plus haut, les cuvettes 14 servent à établir la liaison entre la partie centrale 1 de l'appareil des fig. 1 et 2 et les rotules 4 servent à positionner les étaux 6.

La partie centrale 1 de l'appareil d'allongement et de compression comprend un cylindre extérieur 20 dont l'une des extrémités 21 est partiellement fermée et présente un alésage axial 22, dans lequel une tige 23 est introduite. La tige 23 est reliée à la cuvette 14 de la rotule 4 par l'intermédiaire d'une collerette 24. L'extrémité 21 du cylindre 20 (voir également fig. 5) présente une fente longitudinale 25 (fig. 1) s'étendant jusqu'à l'alésage 22 et permettant de serrer l'extrémité 21 du cylindre 20 entre la tige 23 à l'aide d'une vis à tête carrée 26 semblable aux vis 11 ou 18 des parties d'extrémité 2 et 3 de l'appareil. En examinant les fig. 1 et 2, on remarque que la tige 23 peut être déplacée axialement dans le cylindre 20 et bloquée à l'aide de la vis 26 dans une position choisie par l'utilisateur entre deux positions limites représentées dans le dessin. Dans la vue en perspective de la fig. 1, la tige 23 est placée dans une première position limite où elle est sortie et permet d'utiliser l'appareil d'allongement sur sa plus grande longueur. Dans la coupe de la fig. 2, la tige 23 est rentrée et permet d'utiliser l'appareil d'allongement sur une plus courte distance. Pour des utilisations particulières, il est évident pour l'homme du métier que l'appareil peut être livré avec des tiges 23 de différentes longueurs. A l'intérieur du cylindre 20 coulisse un demi-cylindre d'avance 27 présentant sur son demi-manteau extérieur un filetage 28 attaqué par une partie filetée correspondante 29 d'un anneau d'avance 30 monté sur le cylindre extérieur 20. En tournant l'anneau 30 qui est retenu latéralement contre les bords 31, 32 d'une rainure excentrique non représentée pratiquée dans le cylindre 20, on fait avancer ou reculer le demi-cylindre fileté 27. L'anneau présente au moins un trou radial 33 agencé pour recevoir une tige permettant de le faire tourner plus facilement. Le demi-cylindre 27 présente sur sa partie centrale une rainure axiale 34 de section rectangulaire au fond de laquelle est fixée une règle graduée 35 permettant de lire l'avance du demi-cylindre 27 par rapport à l'extrémité libre 36 du cylindre extérieur 20, le début de la graduation de la règle coïncide avec la cuvette 14 de la rotule 4. Le filetage 28 du demi-cylindre 27 et le filetage 29 de l'anneau 30 sont exécutés de manière à ce qu'un tour complet de l'anneau 30 corresponde à une avance de 2 mm du demi-cylindre fileté 27.

Le demi-cylindre fileté 27 glisse contre un deuxième demi-cylindre 37 visible dans les fig. 3 et 4, le demi-cylindre 37 étant introduit dans le cylindre extérieur 20 et étant agencé pour effectuer une fonction de blocage de la partie centrale 1 de l'appareil et une fonction de rattrapage de jeu par rapport à l'ensemble formé par le cylindre fileté 27 et le cylindre extérieur 20. Le demi-cylindre 37 est comme le demi-cylindre 27 introduit à l'intérieur du cylindre extérieur 20. Comme représenté dans la fig. 2 du dessin, les demi-cylindres 27 et 37 ont une longueur sensiblement égale à celle du cylindre extérieur 20. Le demi-cylindre de blocage et de rattrapage de jeu 37 présente sur toute sa longueur une gorge circulaire axiale 38 faisant face à la rainure 34 du demi-cylindre 27. La gorge 38 et la rainure 34 sont réalisées de manière à laisser entre les deux demi-cylindres 27 et 37 un passage libre pour la tige 23, ceci quelle que soit la position du cylindre d'avance 27. Le demi-cylindre 37 présente d'autre part sur environ la moitié de sa longueur une fente 39 (voir fig. 3 et 4) séparant le demi-cylindre en deux parties sensiblement égales. On remarque dans la fig. 4 que la partie du demi-cylindre 37 située à droite de la fente 39 comprend un alésage fileté 40 destiné à recevoir une vis de serrage 41. L'alésage fileté 40 se prolonge en 42 dans le cylindre extérieur 20, de sorte que la vis 41 est introduite à travers le cylindre 20 après que le demi-cylindre 37 ait été mis en place. La vis 41 est ensuite vissée dans l'alésage 42 et 40 jusqu'à ce qu'elle vienne buter contre la partie gauche du demi-cylindre 37 situé à gauche de la fente 39. L'homme du métier comprendra aisément que si l'on continue à visser la vis 41 après que celle-ci soit venue buter contre la partie gauche du demi-cylindre 37, on écartera de plus en plus les deux parties du demi-cylindre 37, qui s'appuyant contre le cylindre extérieur 20 sera déplacé vers le bas. En réglant convenablement la position de la vis 41, on pourra régler le jeu existant entre le demi-cylindre d'avance 27 et le cylindre extérieur 20. Dans la fig. 2, on remarque qu'il est prévu deux vis 41 identiques, placées à distance l'une de l'autre. Les deux vis permettront de rattraper le jeu du demi-cylindre d'avance 27 dans le cylindre extérieur 20, ces deux éléments ainsi que le demi-cylindre 37 pouvant alors être fabriqués sans précaution particulière, une grande précision d'usinage ne se justifiant plus. Les vis 41 étant en place, les alésages 42 dans le cylindre 20 sont fermés à l'aide de vis capuchons 43 (voir fig. 1).

A leur extrémité libre, les deux parties du demi-cylindre 37 sont utilisées pour effectuer une fonction de blocage du cylindre d'avance 27. Comme représenté dans les fig. 1, 2 et 3, la partie d'extrémité du cylindre 37 placée à droite de la rainure 39 présente un alésage 44 prolongé dans le cylindre extérieur 20, l'alésage 44 recevant une vis à tête carrée 45 butant contre la partie gauche (fig. 3) du demi-cylindre 37. Le serrage de la vis 45 permet de bloquer le demi-cylindre d'avance 27, l'élargissement de la fente 39 provoqué par la vis 45 occasionnant le déplacement vers le bas (fig. 3) du demi-cylindre 37 qui glisse contre les parois intérieures du cylindre 20 et par conséquent une action de coïncement du cylindre d'avance 27.

L'appareil d'allongement et de compression qui vient d'être décrit en regard des fig. 1 à 5 fonctionne comme suit:

Supposons que l'on désire écarter deux fragments d'os d'une cassure et que pour ce faire on décide d'utiliser un fixateur externe. On met alors préalablement en place dans les fragments d'os les fiches d'acier non représentées destinées à venir se placer entre les mâchoires 7 et 8 des étaux 6. Les fiches d'acier non représentées sont mises en place dans les étaux 6 et les vis à tête carrée 11 sont bloquées. La partie centrale 1 et les parties d'extrémité 2 avec les rotules 4 sont placées sur les tiges 5 des étaux 6, les vis 26 et 45 étant débloquées. Les vis à ailette 17 permettant le blocage des rotules sont évidemment également débloquées. Lorsque l'appareil est en place, on bloque les deux vis à ailette 17 des rotules et la vis à tête carrée 26 (réglage grossier en longueur). Avant la mise en place de la partie centrale 1, on aura préalablement réglé le rattrapage de jeu à l'aide des vis 41, de manière à ce que le demi-cylindre d'avance 37 coulisse dans le cylindre extérieur sans jeu et avec un minimum de frottement, et on aura placé le demi-cylindre d'avance 27 dans sa position désirée. Comme déjà mentionné plus haut, la partie centrale 1 de l'appareil et les rotules pourront être mises en place sans difficulté, même dans le cas où les fiches vissées dans les fragments d'os ne sont pas situées dans le même plan. En effet, les rotules 4 permettent de monter la partie centrale 1 sur les tiges 5 quelle que soit la position de ces tiges et l'angle qu'elles peuvent former entre elles. L'homme du métier comprendra aisément que la partie centrale 1 peut être montée pour relier des étaux 6 qui non seulement ne sont pas dans le même plan, mais dont les tiges supports 5 regardent par exemple dans des directions opposées l'une à l'autre.

Les vis à ailette 17 ainsi que la vis 26 étant bloquées, on peut exercer sur les deux fragments d'os un allongement ou une compression (réglage fin) en tournant simplement l'anneau 30 dans un sens ou dans l'autre. Etant donné qu'un tour d'anneau correspond à un allongement ou à une compression de 2 mm et que tout jeu est rattrapé grâce au demi-cylindre 27, il est possible de régler l'avance du demi-cylindre 27 avec une précision pouvant aller au dixième de mm. L'appareil présente encore le très grand avantage de pouvoir effectuer une compression ou un allongement sans que la position des éléments de l'appareil soit modifiée. Il est encore visible que deux ou plusieurs appareils semblables peuvent être utilisés ensemble. En particulier, une paire d'appareils peut être montée aux extrémités des fiches, lorsque celles-ci sont utilisées comme fiches transfixiantes, c'est-à-dire comme fiches traversant des fragments qu'elles sont sensées mettre sous tension.

Dans la fig. 6 représentant une variante de la cuvette 14 placée au bout de la tige 23 et portant les pièces latérales 12 et 13 de l'élément de blocage orientable 4 maintenues dans la rainure circulaire 46, il a été prévu de monter un dynamomètre de mesure de la force appliquée sur les fiches maintenues dans les étaux 6 lors de l'allongement ou de la compression des parties squelettiques entre lesquelles l'appareil est monté. Dans ce but, la cuvette 14 est formée de deux parties 47 et 48, la partie 48 portant la rainure 46. La partie 47 solidaire de la tige 23 comprend une rainure circulaire 49 de coupe rectangulaire dans laquelle vient se loger  une nervure circulaire de coupe rectangulaire correspondante 50 de la pièce 48. Pour des raisons d'étanchéité, il a été prévu sur un des côtés de la rainure 49, respectivement de la nervure 50 un "O-ring" 51, respectivement 52 placé dans des rainures correspondantes. Les deux pièces 47 et 48 formant la cuvette 14 sont maintenues par une vis 53 introduite dans un alésage 54 de la pièce 47 et dont la tête s'appuye sur une butée circulaire 55 de la pièces 48. Dans la nervure 49 est placée une jauge de contrainte ou un élément à quartz piézo-électrique 56 dont les électrodes de mesure sont représentées en 57 et 58 et sont protégées par un capuchon 59.

L'homme du métier comprendra aisément l'intérêt de la jauge de contrainte placée dans la cuvette 14. Il suffit en effet de brancher un appareil de mesure étalonné aux électrodes 57, 58 après avoir préalablement enlevé le capuchon de protection pour pouvoir mesurer la force de compression ou d'allongement que l'on applique avec l'appareil. Comme il a été prévu des joints "O-ring" 51, 52 et un capuchon de protection 59, la jauge de contrainte ou le piézoquartz est placé à l'intérieur d'une enceinte étanche et la cuvette 14 de la fig. 6 peut donc être désinfectée ou mise dans un autoclave de stérilisation avec le reste de l'appareil sans subir aucun dommage. Il est évident que la jauge de contrainte ou le piézoquartz monté dans la cuvette 14 de la fig. 6 peut être placé à un autre endroit de l'appareil des fig. 1 à 5, par exemple entre le cylindre extérieur 20 formant l'enveloppe de la partie centrale de l'appareil et l'anneau 30. En effet, si l'on place deux jauges de contrainte de part et d'autre de l'anneau 30 entre celui-ci et la butée latérale du cylindre 20 lui servant
d'appui sur chacun de ses côtés, on pourra mesurer sans
difficulté toute force d'allongement et de compression,
étant donné que cette force est appliquée directement
par ledit anneau 30.

D'autre part, la jauge de contrainte ou le
piézoquartz peut être remplacé si nécessaire par un
dispositif plus simple et moins onéreux, par exemple
un simple dynamomètre à ressort qui pourra être monté
à tout endroit adéquat de l'appareil des fig. 1 à 5,
par exemple sur ou dans la tige 23 (fig. 1) ou encore
entre le cylindre 20 et l'anneau 30. On peut par exemple utiliser dans ce cas de simples rondelles en forme
de cuvettes ou de troncs de cône en acier inox susceptibles d'être comprimées et placées de chaque côté de
l'anneau entre celui-ci et la butée pratiquée dans
le cylindre 20. Un dispositif micrométrique permettra
de lire la déformation subie par la rondelle et par
conséquent la force d'allongement ou de compression.
Les rondelles et dispositifs micrométriques mentionnés
ci-dessus sont parfaitement connus et disponibles sur
le marché.

L'appareil représenté dans les fig. 1 à 5
du dessin à une longueur totale allant de 220 à 440 mm
suivant la position du demi-cylindre d'avance 27. Sa
partie centrale comprend un cylindre ayant une longueur
de 130 mm. Il est évident que l'on peut aller au-delà
de 400 mm, en choisissant des tiges 23 de plus grandes
longueurs. L'appareil peut être réalisé en acier inox
ou en métal léger, notamment en un alliage d'aluminium
ou de titane.

L'appareil qui vient d'être décrit présente les avantages suivants:

- il permet d'effectuer sur des fragments d'os un allongement ou une compression selon un axe avec une précision supérieure à 1/10 de mm et sur une distance de 80 mm, tout réglage d'allongement ou de compression étant effectué selon cet axe sans intervenir sur le positionnement des autres éléments de fixation de l'appareil,

- il permet grâce aux éléments de blocage orientables ou rotules 4 d'assembler des étaux porte-fiches placés dans n'importe quelle direction, l'appareil pouvant être placé sur les fiches et bloqué sur ces dernières sans exercer aucune action (tension ou torsion) dans une direction ou un sens non désiré,

- il offre après sa mise en place une rigidité totale,

- il ne présente pas de jeu lors du réglage fin, étant donné ses moyens de rattrapage de jeu,

- lorsqu'il est dans la position requise d'allongement ou de compression après son réglage fin à l'aide de l'anneau 30, il peut être bloqué dans cette position,

- il présente une possibilité de réglage grossier grâce à la tige 23 et à la vis de serrage 26 lors de sa mise en place,

- il permet de doser exactement la force d'allongement ou de compression appliquée aux parties squelettiques dans le cas où l'on utilise la variante de l'appareil avec dynamomètre ou jauge de contrainte.

Bien que l'appareil décrit en regard des fig. 1 à 5 du dessin ait été représenté en liaison avec des étaux destinés à maintenir des fiches introduites dans des fragments d'os à maintenir, il est évident qu'il peut être utilisé pour d'autres buts. Par exemple, il peut être utilisé en combinaison avec tout fixateur externe et ses rotules peuvent prendre appui non seulement sur des tiges supportant des étaux, mais sur toute tige ou moyen d'assemblage d'un fixateur pour fixation squelettique externe.

Les éléments de blocage orientables ou rotules formant ses parties d'extrémité peuvent même être remplacées par des moyens d'assemblage semblables ou équivalents.

REVENDICATIONS

1. Appareil d'allongement et de compression pour fixation squelettique externe, présentant des moyens d'assemblage de fiches transcutanées ou d'éléments reliés à ces fiches et des moyens d'allongement et de compression placés entre les moyens d'assemblage, caractérisé en ce que les moyens d'allongement et de compression comprennent au moins deux pièces coulissantes l'une par rapport à l'autre selon un axe commun, l'une des pièces présentant un filetage attaqué par un filetage correspondant d'un anneau d'avance maintenu dans le sens de l'axe d'avance sur les moyens d'allongement et de compression, des moyens de rattrapage de jeu étant prévus entre les deux pièces.

2. Appareil selon la revendication 1, caractérisé en ce que les deux pièces coulissantes comprennent un demi-cylindre d'avance fileté et un demi-cylindre d'appui placés face à face, le tout étant maintenu à l'intérieur d'un cylindre formant une enveloppe extérieure.

3. Appareil selon la revendication 2, caractérisé en ce que le demi-cylindre d'appui comprend une fente s'étendant sur une partie de sa longueur et séparant le demi-cylindre en deux parties, au moins une vis de rattrapage de jeu et une vis de blocage étant introduites dans des alésages filetés correspondants prévus dans une des parties du demi-cylindre et débouchant sur l'autre partie.

4. Appareil selon la revendication 2, caractérisé en ce que les deux demi-cylindres d'avance et d'appui présentent respectivement une rainure de section rectangulaire et une gorge de section circulaire placées adjacentes, de manière à laisser un vide sur l'axe commun des deux demi-cylindres, une règle graduée étant placée au fond de la rainure du cylindre d'avance.

5. Appareil selon la revendication 2, caractérisé en ce que les moyens d'assemblage avec les fiches transcutanées ou avec les éléments reliés à ces fiches sont reliés à une extrémité du demi-cylindre d'avance, respectivement à une extrémité opposée du cylindre extérieur.

6. Appareil selon les revendications 4 et 5, caractérisé en ce qu'une tige de réglage de longueur est intercalée entre les moyens d'assemblage et l'extrémité du cylindre extérieur présentant des moyens de blocage de la tige qui est agencée pour venir se placer entre la gorge et la rainure des deux demi-cylindres d'allongement.

7. Appareil selon la revendication 5, caractérisé en ce que les moyens d'assemblage comprennent deux éléments de blocage orientables agencés pour bloquer une tige dans toutes directions.

8. Appareil selon la revendication 7, caractérisé en ce que les éléments de blocage orientables présentent deux pièces latérales serrant entre elles deux plaquettes circulaires tournant entre les pièces latérales et dont les surfaces adjacentes présentent chacune une rainure diamétrale apte à serrer une tige, les pièces latérales étant retenues à une de leurs extrémités dans une gorge d'une cuvette reliée à l'extrémité de la pièce coulissante, respectivement
du cylindre externe, les deux autres extrémités des
pièces latérales étant traversées par une vis de serrage.

9. Appareil selon la revendication 7, caractérisé en ce que les tiges bloquées par les éléments
de blocage orientables sont montées sur une des mâchoires d'un étau de fixation des fiches transcutanées
maintenant les fragments d'os.

10. Appareil selon la revendication 1, caractérisé en ce qu'il comprend un dispositif de mesure
agencé pour mesurer la force de compression ou d'allongement appliquée entre les moyens d'assemblage des
fiches transcutanées.

11. Appareil selon la revendication 10, caractérisé en ce que le dispositif de mesure est un
dynamomètre.

12. Appareil selon la revendication 10, caractérisé en ce que le dispositif de mesure est une
jauge de contrainte ou un piezo-quartz.

13. Appareil selon la revendication 1, caractérisé en ce qu'il est réalisé en acier inox.

14. Appareil selon la revendication 1, caractérisé en ce qu'il est réalisé en un alliage de
titane ou d'aluminium.

0024256

Fig. 1.

Fig. 6

Fig. 2

Fig. 3

Fig. 4

Fig. 5